# EUROPÄISCHE PATENTSCHRIFT

(11) **EP 2 361 078 B1**
(45) Veröffentlichungstag und Bekanntmachung des Hinweises auf die Patenterteilung: **30.01.2019**
(21) Anmeldenummer: 09795988.6
(22) Anmeldetag: 21.12.2009
(51) Int. Cl.: A61K 8/06, A61K 8/28, A61K 8/37, A61K 8/39, A61K 8/893, A61K 8/894, A61Q 15/00

(54) **TRANSPARENTE SCHWEIßHEMMENDE GELE**
TRANSPARENT ANTIPERSPIRANT GELS
GELS TRANSPARENTS ANTI-TRANSPIRANTS

(30) Priorität: 22.12.2008 DE 102008064198
(43) Veröffentlichungstag der Anmeldung: 31.08.2011
(73) Patentinhaber: Henkel AG & Co. KGaA, 40589 Düsseldorf (DE)
(72) Erfinder: BANOWSKI, Bernhard, 40597 Düsseldorf (DE); BUSE, Nadine, 40724 Hilden (DE); CLAAS, Marcus, 40723 Hilden (DE)
(86) Internationale Anmeldenummer: PCT/EP2009/067623
(87) Internationale Veröffentlichungsnummer: WO 2010/072705

(56) Entgegenhaltungen:
- EP-A1- 1 057 473
- WO-A1-2005/063189
- WO-A1-2006/046945
- WO-A2-2009/083545
- CA-A1- 2 054 478
- DE-A1-102007 059 297
- US-A1- 2004 241 196
- Technical Data Sheet relating to INCI PEG-7 Glyceryl Cocoate

## Beschreibung

Gegenstand der vorliegenden Anmeldung sind transparente schweißhemmende Zusammensetzungen in Form einer Wasser-in-Öl-Emulsion, die zur Verbesserung der schweißhemmenden Wirkung eine ausgewogene Mischung aus hoch wirksamen Antitranspirant-Wirkstoffen, ausgewählten Ölkomponenten, Emulgatoren und Polyethylenglycolethern von Alkanolen enthalten.

### Stand der Technik

Schweißhemmende Zusammensetzungen in Form einer Wasser-in-Öl-Emulsion sind im Stand der Technik bereits bekannt. WO 92/05767 A1 offenbart transparente schweißhemmende Gele in Form einer Wasser-in-Öl-Emulsion auf der Basis von Cyclomethiconen und emulgierenden Ethylenoxid-Propylenoxid-substituierten Polydimethylsiloxanen. WO 96/06594 A1 offenbart transparente schweißhemmende Wasser-in-Öl-Zusammensetzungen, die flüchtige Siliconöle und/oder flüchtige Kohlenwasserstofföle und die Silicon-freien Wasser-in-Öl-Emulgatoren Laureth-1 oder Laureth-4 enthalten. WO 98/17238 A1 offenbart schweißhemmende Stifte in Form einer Wasser-in-Öl-Emulsion auf der Basis von mit Alkylresten endständig disubstituierten Silicon-freien Wasser-in-Öl-Emulgatoren, ohne das Problem der Transparenz zu behandeln. WO 00/067888 A1 offenbart dünnflüssige W/O-Emulsionen mit einer Viskosität von maximal 5000 mPas, mindestens 75 Gew.- % Wasserphase, maximal 20 Gew.-% Lipide, Emulgatoren und weitere lipophile Bestandteile, wobei die Ölphase eine Gesamtpolarität zwischen 20 und 30 mN/m aufweist und frei ist von Siliconen, stabilisiert durch mit Alkylresten endständig disubstituierten Silicon-freien Wasser-in-Öl-Emulgatoren. Auch diese Schrift beschäftigt sich nicht mit dem Problem der Transparenz. DE 10 2007 059 297 A1 offenbart transparente schweißhemmende Zusammensetzungen in Form einer Wasser-in-Öl-Emulsion, welche einen geringen Gehalt an Cyclotetra- und Cyclopentasiloxan, jedoch einen Gehalt an Cyclohexasiloxan von 5 bis 25 Gew.-% aufweisen. WO2005/063189 A1 offenbart wasserfreie und silikonfreie schweißhemmende Zusammensetzungen, welche eine bestimmte Ölphase aufweisen, um ein Absetzen des partikelförmigen schweißhemmenden Wirkstoffs zu vermeiden. US 2004/0241196 A1 beschreibt transparente schweißhemmende Gele in Form von Wasser-in-Öl-Emulsionen, welche propoxylierte Alkohole als Lösungsvermittler für Parfümöle enthalten. Schließlich offenbart WO 2009/083545 A2 transparente schweißhemmende Zusammensetzungen in Form von Wasser-in-Öl-Emulsionen, bei welchen der schweißhemmende Wirkstoff in der wässrigen Phase gelöst ist.

### Aufgabe

Transparente Gele in Form einer Wasser-in-Öl-Emulsion erfreuen sich beim Verbraucher großer Beliebtheit. Die bekannten treibgasfreien W/O-Gele auf der Basis von Cyclomethiconen ergeben bei der Applikation auf die Haut ein frisches und gleichzeitig pflegendes Gefühl. Gleichzeitig zeigen sie direkt nach der Applikation zunächst keine sichtbaren Rückstände. Diese - anfangs - weitgehend rückstandsfreie Applikation, die vom Verbraucher sehr geschätzt wird, wird für ihn auch durch die Transparenz der Zusammensetzung visualisiert.

Für optimale Transparenz muss der Brechungsindex der Ölphase und der Wasserphase innerhalb von etwa 0,001 oder besser und bevorzugt innerhalb von etwa 0,0004 aufeinander abgestimmt sein. Bei der Festlegung eines Bestandteils, beispielsweise der besonders effektiven schweißhemmenden Aluminium-Zirconium-Verbindungen, die einen relativ hohen Brechungsindex der wässrigen Phase mit sich bringen, ist man also in der Wahl der übrigen Bestandteile eingeschränkt. Ein Problem der bekannten Gele ist ihr hoher Gehalt an Cyclomethicone. Bei den handelsüblichen Cyclomethiconen unterscheidet man vor allem Cyclotetrasiloxan, Cyclopentasiloxan und Cyclohexasiloxan. Cyclotetrasiloxan, dessen Schmelzpunkt mit-11°C ungewöhnlich hoch liegt, kann in den für ein Wasser-in-Öl-Emulsionsgel typischen höheren Einsatzmengen zu Problemen bei der Lagerstabilität führen. Außerdem sieht man heutzutage aus toxikologischen Gründen weitgehend von einem Einsatz des Cyclotetrasiloxans ab. Die üblichen Cyclomethicone-Handelsprodukte sind nahezu frei von Cyclotetrasiloxan. Dennoch ist die Substanzklasse der Cyclomethicone aufgrund selbst des Spurengehalts an Cyclotetrasiloxan ein problematischer Rohstoff; Cyclosiloxane im Allgemeinen werden daneben wegen ihrer Persistenz in der Umwelt diskutiert. Andererseits weisen die Cyclomethicone hervorragende Gebrauchseigenschaften auf, so dass ihr Ersatz ausgesprochen schwierig ist. Cyclomethicone sind relativ flüchtige Ölkomponenten. Aus diesem Grund werden sie gern in Kosmetika, insbesondere in Antitranspirantien, eingesetzt, da sie helfen, das Problem des Anschmutzens der Kleidung zu lösen. Andererseits bilden Antitranspirantien mit einem zu hohen Anteil an flüchtigem Cyclomethicone nach dem Antrocknen, das heißt erst einige Zeit nach der Applikation, auf der Haut weiße Rückstände, die schlecht auf der Haut haften und herunterrieseln können, was von vielen Verbrauchern als unangenehm empfunden wird.

Eine Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen sind.

Eine weitere Aufgabe der vorliegenden Anmeldung war es, transparente schweißhemmende Zusammensetzungen in Form einer Wasser-in-Öl-Emulsion bereitzustellen, die einen hohen Gehalt an dispergierter wässriger Phase, vorzugsweise mindestens 75 Gew.-%, aufweisen. Da die handelsüblichen schweißhemmenden Wirkstoffe wasserlöslich sind, befinden sie sich in der wässrigen Phase. Eine schnelle Freisetzung des Wirkstoffes kann dabei durch einen hohen Gehalt an wässriger Phase unterstützt bzw. durch die Wahl der Emulgatoren und Zusätze in der wässrigen Phase deutlich verbessert werden.

Eine weitere Aufgabe der vorliegenden Anmeldung war es, transparente schweißhemmende Zusammensetzungen in Form einer Wasser-in-Öl-Emulsion bereitzustellen, die eine gelartige Konsistenz mit mittlerer bis hoher Viskosität aufweisen und mittels einer Applikationskugel (Roll-on) oder einer stiftähnlichen Hülse (Cremespender) aufgetragen werden können.

Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen sind und eine gute Freisetzung des schweißhemmenden Wirkstoffs zeigen.

Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen und ausreichend lagerstabil sind.

Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen und ausreichend lagerstabil sind und eine gute Freisetzung des schweißhemmenden Wirkstoffs zeigen.

Überraschend wurde festgestellt, dass Zusammensetzungen gemäß Anspruch 1 die gestellten Aufgaben hervorragend lösen.

Gegenstand der vorliegenden Anmeldung sind transparente schweißhemmende Zusammensetzungen in Form einer Wasser-in-Öl-Emulsion, enthaltend
a) 10 - 20 Gew.-% äußere Ölphase, enthaltend
   a.i) mindestens einen symmetrischen, unsymmetrischen oder cyclischen Ester der Kohlensäure mit linearen oder verzweigten C₆ - C₂₂-Alkanolen,
   a.ii) mindestens ein Anlagerungsprodukt von 1 - 14 Propylenoxid-Einheiten an ein- oder mehrwertige C₃₋₁₆-Alkanole sowie
   a.iii) Cyclomethicone in einer Gesamtmenge von 0 bis maximal 3 Gew.-%,
b) 75 - 90 Gew.-% dispergierte wässrige Phase, darin
   b.i) mindestens eine Verbindung, ausgewählt aus wasserlöslichen mehrwertigen C₂-C₉-Alkanolen mit 2 - 6 Hydroxylgruppen und wasserlöslichen Polyethylenglycolen mit 3 - 20 Ethylenoxid-Einheiten, in einer Gesamtmenge von 20 - 60 Gew.-%,
   b.ii) 5 - 40 Gew.-% (USP) mindestens einer schweißhemmenden Aluminium-Zirkonium-Verbindung,
   b.iii) freies (= nicht molekular gebundenes) und molekular gebundenes Wasser in einer Gesamtmenge von 5 - 40 Gew.-%, bevorzugt 6 - 30 Gew.-%, besonders bevorzugt 7 - 22 Gew.-%,
c) mindestens einen Silicon-basierten Wasser-in-Öl-Emulgator mit mindestens einem Alkylsubstituenten R mit 4 - 20 Kohlenstoffatomen,
d) keinen Silicon-freien Wasser-in-Öl-Emulgator, wobei der Begriff Silicon-freier Wasser-in-Öl-Emulgator Laureth-2, PEG-6 Caprylic/Capric Glycerides, PPG-15 Stearyl Ether, PEG-7 Glyceryl Cocoate sowie einer Mischung aus Polyglyceryl-4 Diisostearate, Polyhydroxystearate und Polyhydroxysebacate einschließt,
e) mindestens einen Polyethylenglycolether eines linearen oder verzweigten C₁₂ - C₂₂-Alkanols mit 20 - 150 Ethylenoxideinheiten,
wobei mindestens 20 Gew.-% der mindestens einen schweißhemmenden Aluminium-Zirkonium-Verbindung, bezogen auf die Gesamtmenge an schweißhemmender Aluminium-Zirkonium-Verbindung, solubilisiert sind in mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten.

Dabei beziehen sich alle Gew.-%-Angaben auf das Gesamtgewicht der Emulsion.

Die erfindungsgemäßen Zusammensetzungen enthalten 10 - 20 Gew.-% äußere Ölphase; bevorzugt beträgt der Gehalt an Ölphase 12 - 18 Gew.-%, besonders bevorzugt 14 - 16 Gew.-%, jeweils bezogen auf das Gesamtgewicht der Emulsion.

Die Bezeichnungen "äußere Ölphase" und "Ölphase" werden in der vorliegenden Anmeldung synonym gebraucht.

Der Silicon-basierte Wasser-in-Öl-Emulgator sowie gegebenenfalls weitere emulgierende Bestandteile werden im Sinne dieser Anmeldung nicht zur Ölphase gezählt. Lipophile Bestandteile, die unter Normalbedingungen nicht flüssig ("Öl"), sondern fest vorliegen, z. B. Wachse, werden im Sinne dieser Anmeldung zur Ölphase gezählt. Parfümöle werden im Sinne dieser Anmeldung zur Ölphase gezählt. Gegebenenfalls enthaltenes Ethanol sowie die mehrwertigen C₂ - C₉-Alkanole mit 2 - 6 Hydroxylgruppen und die wasserlöslichen Polyethylenglycole mit 3 - 20 Ethylenoxid-Einheiten werden im Sinne dieser Anmeldung nicht zur Ölphase, sondern zur Wasserphase gezählt, auch wenn sie zumindest teilweise öllösliche Eigenschaften aufweisen.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die Ölphase enthält zwingend eine Kombination aus mindestens einem symmetrischen, unsymmetrischen oder cyclischen Ester der Kohlensäure mit linearen oder verzweigten C₆ - C₂₂-Alkanolen und mindestens einem Anlagerungsprodukt von 1 - 14 Propylenoxid-Einheiten an ein- oder mehrwertige C₃₋₁₆-Alkanole.

Überraschend wurde festgestellt, dass diese Kombination aus Ölkomponenten einen hervorragenden Ersatz für den Cyclomethicone-Anteil in bekannten schweißhemmenden W/O-Emulsionen darstellt. Diese Ölkombinationen unterstützen in überraschender Weise die Wirkstofffreisetzung des schweißhemmenden Wirkstoffs. Weiterhin bieten diese Öle ein ähnlich angenehmes Hautgefühl wie Cyclomethicone. Weiterhin lassen sich mit diesen Ölen aufgrund ihrer Brechungsindices sehr gut hoch-transparente W/O-Emulsionen herstellen.

Bevorzugte Ölkomponenten, die symmetrische, unsymmetrische oder cyclische Ester der Kohlensäure mit linearen oder verzweigten C₆ - C₂₂-Alkanolen darstellen, sind Di-n-hexylcarbonat, Di-n-heptylcarbonat, Di-n-octylcarbonat, das unter der INCI-Bezeichnung Dicaprylyl Carbonate beispielsweise als Handelsprodukt Cetiol® CC von Cognis erhältlich ist, weiterhin Di-(2-ethylhexyl)carbonat, das unter der INCI-Bezeichnung Diethylhexyl Carbonate beispielsweise als Handelsprodukt Tegosoft® DEC von Degussa-Evonik erhältlich ist, weiterhin Di-n-nonylcarbonat, Di-n-decylcarbonat, Di-n-laurylcarbonat, Di-n-myristylcarbonat, Di-(C₁₄-C₁₅)-alkylcarbonat, Di-n-cetylcarbonat, Di-isocetylcarbonat, Di-n-stearylcarbonat, Di-isostearylcarbonat, Di-arachylcarbonat und Di-behenylcarbonat als symmetrisch substituierte Carbonate, sowie unsymmetrisch substituierte Carbonate, wie n-Hexyl-n-octylcarbonat, n-Hexyl-2-ethylhexylcarbonat und n-Octyl-n-decylcarbonat. Cyclische Ester der Kohlensäure sind erhältlich durch Umesterung von Dimethylcarbonat oder Diethylcarbonat mit zwei- oder mehrwertigen Alkanolen.

Besonders bevorzugte erfindungsgemäße Zusammensetzungen sind dadurch gekennzeichnet, dass der mindestens eine symmetrische, unsymmetrische oder cyclische Ester der Kohlensäure mit linearen oder verzweigten C₆ - C₂₂-Alkanolen ausgewählt ist aus Di-n-octylcarbonat, Di-(2-ethylhexyl)carbonat, Di-(C₁₄-C₁₅)-alkylcarbonat, Di-n-decylcarbonat und Di-n-laurylcarbonat.

Außerordentlich bevorzugt sind Di-n-octylcarbonat, das bei 21 °C einen Brechungsindex n_{D} von 1,435 - 1,436 aufweist, sowie Di-2-ethylhexylcarbonat.

Bevorzugte erfindungsgemäße Zusammensetzungen sind dadurch gekennzeichnet, dass mindestens ein symmetrischer, unsymmetrischer oder cyclischer Ester der Kohlensäure mit linearen oder verzweigten C₆ - C₂₂-Alkanolen in einer Gesamtmenge von 8 - 18 Gew.-%, besonders bevorzugt 10 - 16 Gew.-%, außerordentlich bevorzugt 12 - 14 Gew.-%, bezogen auf die gesamte Zusammensetzung, enthalten ist.

Weitere besonders bevorzugte erfindungsgemäße Zusammensetzungen sind dadurch gekennzeichnet, dass die hydrophobe Ölphase ohne die Emulgatoren zu mindestens 60 Gew.-%, bevorzugt zu mindestens 70 Gew.-%, besonders zu mindestens 80 Gew.-%, außerordentlich bevorzugt zu mindestens 90 Gew.-%, aus mindestens einem symmetrischen, unsymmetrischen oder cyclischen Ester der Kohlensäure mit linearen oder verzweigten C₆ - C₂₂-Alkanolen besteht.

Weitere besonders bevorzugte erfindungsgemäße Zusammensetzungen sind dadurch gekennzeichnet, dass die hydrophobe Ölphase ohne die Emulgatoren zu mindestens 60 Gew.-%, bevorzugt zu mindestens 70 Gew.-%, besonders zu mindestens 80 Gew.-%, außerordentlich bevorzugt zu mindestens 90 Gew.-%, aus Di-n-octylcarbonat besteht.

Weitere besonders bevorzugte erfindungsgemäße Zusammensetzungen sind dadurch gekennzeichnet, dass die hydrophobe Ölphase ohne die Emulgatoren zu mindestens 60 Gew.-%, bevorzugt zu mindestens 70 Gew.-%, besonders zu mindestens 80 Gew.-%, außerordentlich bevorzugt zu mindestens 90 Gew.-%, aus Di-2-ethylhexylcarbonat besteht.

Weitere besonders bevorzugte erfindungsgemäße Zusammensetzungen sind dadurch gekennzeichnet, dass die hydrophobe Ölphase ohne die Emulgatoren zu mindestens 60 Gew.-%, bevorzugt zu mindestens 70 Gew.-%, besonders zu mindestens 80 Gew.-%, außerordentlich bevorzugt zu mindestens 90 Gew.-%, aus Di-n-octylcarbonat und Di-2-ethylhexylcarbonat besteht.

Als weitere obligatorische Ölkomponente enthalten die erfindungsgemäßen Zusammensetzungen mindestens ein Anlagerungsprodukt von 1 - 14 Propylenoxid-Einheiten an ein ein- oder mehrwertiges C₃₋₁₆-Alkanol, insbesondere an n-Butanol-1, n-Pentanol-1, n-Hexanol-1, n-Heptanol-1, n-Octanol-1, n-Decanol-1, n-Decan-1,10-diol, Laurylalkohol, Myristylalkohol und Cetylalkohol. Erfindungsgemäß bevorzugt sind Anlagerungsprodukte von 1 - 8 Propylenoxid-Einheiten an ein ein- oder mehrwertiges C₃₋₁₆-Alkanol. Weiterhin erfindungsgemäß bevorzugt sind Anlagerungsprodukte von 1 - 8 Propylenoxid-Einheiten an ein einwertiges C₃₋₁₆-Alkanol. Weiterhin erfindungsgemäß bevorzugt sind Anlagerungsprodukte von 2 - 3 Propylenoxid-Einheiten an ein einwertiges C₃₋₁₆-Alkanol. Weiterhin erfindungsgemäß bevorzugt sind Anlagerungsprodukte von 1 - 8 Propylenoxid-Einheiten an ein einwertiges C₁₀₋₁₄-Alkanol, insbesondere an n-Decanol-1, Laurylalkohol und Myristylalkohol. Weiterhin erfindungsgemäß bevorzugt sind Anlagerungsprodukte von 2 -3 Propylenoxid-Einheiten an ein einwertiges C₁₀₋₁₄-Alkanol, insbesondere an n-Decanol-1, Laurylalkohol und Myristylalkohol.

Besonders bevorzugt sind PPG-2-Myristylether, PPG-3-Myristylether (Witconol® APM), PPG-4-Myristylether, PPG-5-Myristylether, PPG-2-Laurylether, PPG-3-Laurylether, PPG-4-Laurylether, PPG-5-Laurylether, PPG-6-Laurylether, PPG-2-Decylether, PPG-3-Decylether, PPG-4-Decylether, PPG-5-Decylether, PPG-6-Decylether, PPG-7-Decylether, PPG-2-Octylether, PPG-3-Octylether, PPG-4-Octylether, PPG-5-Octylether, PPG-6-Octylether, PPG-7-Octylether, PPG-8-Octylether, PPG-2-Hexylether, PPG-3-Hexylether, PPG-4-Hexylether, PPG-5-Hexylether, PPG-6-Hexylether, PPG-7-Hexylether, PPG-8-Hexylether und PPG-9-Hexylether.

Ebenfalls gut geeignet, wenn auch weniger bevorzugt, sind PPG-4-Butylether, PPG-5-Butylether, PPG-6-Butylether, PPG-7-Butylether, PPG-8-Butylether, PPG-9-Butylether, PPG-10-Butylether, PPG-11-Butylether, PPG-12-Butylether, PPG-13-Butylether und PPG-14-Butylether (Ucon® Fluid AP).

Es wurde überraschend festgestellt, dass mit einem PPG-Anlagerungsprodukt wie PPG-15-Stearylether (Arlamol® E) nicht die gleichen guten Effekte in Bezug auf die Freisetzung des schweißhemmenden Wirkstoffs erzielt werden konnten wie mit den Anlagerungsprodukten von 1 - 14 Propylenoxid-Einheiten an ein- oder mehrwertige C₃₋₁₆-Alkanole. Erfindungsgemäß besonders bevorzugt sind PPG-3-Myristylether und PPG-2-Myristylether; außerordentlich bevorzugt ist PPG-3-Myristylether. Erfindungsgemäß außerordentlich bevorzugt sind Kombinationen aus Di-n-octylcarbonat und PPG-3-Myristylether, Di-2-ethylhexylcarbonat und PPG-3-Myristylether, Di-n-octylcarbonat und PPG-2-Myristylether, sowie Di-2-ethylhexylcarbonat und PPG-2-Myristylether.

Das mindestens eine Anlagerungsprodukt von 1 - 14 bevorzugt 1 - 8, Propylenoxid-Einheiten an ein ein- oder mehrwertiges C₃₋₁₆-Alkanol, bevorzugt an ein einwertiges C₁₀₋₁₄-Alkanol, ist bevorzugt in einer Gesamtmenge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten.

Weitere erfindungsgemäß bevorzugte schweißhemmende Zusammensetzungen sind dadurch gekennzeichnet, dass PPG-3-Myristylether in einer Menge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten ist. Weitere erfindungsgemäß bevorzugte schweißhemmende Zusammensetzungen sind dadurch gekennzeichnet, dass PPG-2-Myristylether in einer Menge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten ist.

Erfindungsgemäß besonders bevorzugte Zusammensetzungen sind dadurch gekennzeichnet, dass die Ölkomponenten, die symmetrische, unsymmetrische oder cyclische Ester der Kohlensäure mit linearen oder verzweigten C₆ - C₂₂-Alkanolen darstellen, in einem Gewichtsverhältnis von 4 - 19 zu den Ölkomponenten, die Anlagerungsprodukte von 1 - 14 Propylenoxid-Einheiten an ein- oder mehrwertige C₃₋₁₆-Alkanole darstellen, vorliegen. Bevorzugt beträgt dieses Gewichtsverhältnis 5 - 15, besonders bevorzugt 6 - 14 und außerordentlich bevorzugt 7 - 13. Es wurde festgestellt, dass mit einem bevorzugten Gewichtsverhältnis besonders gute Effekte in Bezug auf die Freisetzung des schweißhemmenden Wirkstoffs erzielt werden konnten.

Die erfindungsgemäßen Zusammensetzungen sind dadurch gekennzeichnet, dass 0 bis maximal 3 Gew.-%, bevorzugt 0 bis maximal 2,5 Gew.-%, besonders bevorzugt 0 bis maximal 2 Gew.-%, außerordentlich bevorzugt 0 bis maximal 1 Gew.-%, Cyclomethicone enthalten sind, jeweils bezogen auf das Gesamtgewicht der Emulsion.

Bevorzugte erfindungsgemäße Zusammensetzungen sind weiterhin dadurch gekennzeichnet, dass lineare Polymethylsiloxane oder Polydimethylsiloxane mit einer kinematischen Viskosität bei 25 °C von 20 cSt und darunter in einer Gesamtmenge von 0 bis maximal 3 Gew.-% bevorzugt 0 bis maximal 2,5 Gew.-%, besonders bevorzugt 0 bis maximal 2 Gew.-%, außerordentlich bevorzugt 0 bis maximal 1 Gew.-%, enthalten sind.

Die erfindungsgemäßen Zusammensetzungen enthalten weiterhin 75 - 90 Gew.-% dispergierte wässrige Phase mit mindestens einem schweißhemmenden Wirkstoff und mindestens einer Verbindung, ausgewählt aus wasserlöslichen mehrwertigen C₂ - C₉-Alkanolen mit 2 - 6 Hydroxyl-gruppen und wasserlöslichen Polyethylenglycolen mit 3 - 20 Ethylenoxid-Einheiten.

Bevorzugt beträgt der Gehalt an wässriger Phase 78 - 88 Gew.-%, besonders bevorzugt 80 - 86 Gew.-%, und außerordentlich bevorzugt 81 - 84 Gew.-%, jeweils bezogen auf das Gesamtgewicht der Emulsion.

Der Gesamtgehalt an freiem (= nicht molekular gebundenes) und molekular gebundenem Wasser beträgt 5 - 40 Gew.-%, bevorzugt 6 - 30 Gew.-%, besonders bevorzugt 7 - 22 Gew.-%, jeweils bezogen auf das Gesamtgewicht der Emulsion. Ein Gesamtgehalt an Wasser von 16, 17, 18, 19 20 und 21 Gew.-%, jeweils bezogen auf die gesamte erfindungsgemäße Zusammensetzung, kann außerordentlich bevorzugt sein.

Unter molekular gebundenem Wasser wird im Sinne der vorliegenden Anmeldung insbesondere Kristallwasser, beispielsweise aus den schweißhemmenden Wirkstoffen, verstanden. "Freies Wasser" ist Wasser, das bei der Emulsionsherstellung als solches zugefügt wird bzw. über die Rohstoffe als nicht molekular gebundenes Wasser eingebracht wird.

Weitere bevorzugte erfindungsgemäße Zusammensetzungen sind dadurch gekennzeichnet, dass 1 - 10 Gew.-%, bevorzugt 2 - 8 Gew.-%, besonders bevorzugt 3 - 7 Gew.-%, außerordentlich bevorzugt 4 - 6 Gew.-%, Ethanol enthalten sind, jeweils bezogen auf das Gesamtgewicht der Emulsion. Ein solcher Ethanolgehalt unterstützt vorteilhaft die Emulsionsstabilität und die Transparenz. Andere, ebenfalls bevorzugte erfindungsgemäße Zusammensetzungen sind dadurch gekennzeichnet, dass sie kein Ethanol enthalten.

Emulgierende Bestandteile werden im Sinne dieser Anmeldung nicht zur wässrigen Phase gezählt.

### Aluminium-Zirkonium-Verbindungen

Die erfindungsgemäßen Zusammensetzungen enthalten 5 - 40 Gew.-%, bevorzugt 10 - 35 Gew.- %, besonders bevorzugt 11 - 28 Gew.-% und außerordentlich bevorzugt 12 - 20 Gew.-%, jeweils bezogen auf das Gesamtgewicht der wasser- und kristallwasserfreien und ligandenfreien Aktivsubstanz (USP) in der Gesamtzusammensetzung mindestens einer schweißhemmenden Aluminium-Zirkonium-Verbindung, wobei mindestens 20 Gew.-% der mindestens einen schweißhemmenden Aluminium-Zirkonium-Verbindung, bezogen auf die Gesamtmenge an schweißhemmender Aluminium-Zirkonium-Verbindung, solubilisiert sind in mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten.

Weitere, ebenfalls besonders bevorzugte erfindungsgemäße Zusammensetzungen sind dadurch gekennzeichnet, dass der mindestens eine Antitranspirant-Wirkstoff in einer Menge von 5 - 40 Gew.-%, bevorzugt 8 - 25 Gew.-%, bevorzugt 10 - 22 und besonders bevorzugt 13 - 20 Gew.-%, enthalten ist, jeweils bezogen auf das Gesamtgewicht der wasser- und kristallwasserfreien und ligandenfreien Aktivsubstanz (USP) in der Gesamtzusammensetzung.

Erfindungsgemäß wird unter Wasserlöslichkeit eine Löslichkeit von wenigstens 5 Gew.-% bei 20 °C unter Normalbedingungen verstanden, das heißt, dass Mengen von wenigstens 5 g des mehrwertigen C₂ - C₉-Alkanols mit 2 - 6 Hydroxylgruppen bzw. des Polyethylenglycols mit 3 - 20 Ethylenoxid-Einheiten in 95 g Wasser bei 20 °C löslich sind.

Erfindungsgemäß bevorzugt sind Aluminium-Zirkonium-Glycol-Komplexe, wobei Aluminium-Zirkonium-Propylenglycol-Komplexe besonders bevorzugt sind. Weiterhin sind Aluminium-Zirkonium-Glycol-Komplexe besonders bevorzugt, die Gegenstand von US 5643558 und US 6010688 sind. Besonders bevorzugte schweißhemmende Wirkstoffe sind auch Komplexe aktivierter schweißhemmender Aluminium-Zirkonium-Verbindungen mit einem mehrwertigen Alkohol, die 20 - 50 Gew.-%, besonders bevorzugt 20 - 42 Gew.-%, aktivierte schweißhemmende Aluminium-Zirkonium-Verbindung und 2 - 16 Gew.-% molekular gebundenes Wasser enthalten, wobei der Rest zu 100 Gew.-% mindestens ein Polyol mit 3 bis 6 Kohlenstoffatome und 3 bis 6 Hydroxyl-Gruppen ist. Propylenglycol, Propylenglycol/ Sorbit-Mischungen und Propylenglycol/Pentaerythrit-Mischungen sind bevorzugte derartige Polyole.

Derartige erfindungsgemäß bevorzugte Komplexe einer aktivierten schweißhemmenden Aluminium-Zirkonium-Verbindung mit einem Polyol sind z. B. offenbart in US 5643558 und US 6245325.

Weitere bevorzugte schweißhemmende Wirkstoffe sind Aluminium-Zirkoniumchlorhydrate, wie Aluminium-Zirkoniumtrichlorhydrat, Aluminium-Zirkoniumtetrachlorhydrat, Aluminium-Zirkoniumpentachlorhydrat, Aluminium-Zirkoniumoctachlorhydrat, Aluminium-Zirkonium-Chlorohydrat-Glycin-Komplexe, wie Aluminium-Zirkoniumtrichlorhydrexglycin, Aluminium-Zirkoniumtetrachlorhydrexglycin, Aluminium-Zirkoniumpentachlorhydrexglycin und Aluminium-Zirkoniumoctachlorhydrexglycin.

Erfindungsgemäß besonders bevorzugt sind weiterhin schweißhemmende Zusammensetzungen, in denen 20 - 100 Gew.-% der mindestens einen schweißhemmenden Aluminium-Zirkonium-Verbindung aus mindestens einem Komplex einer schweißhemmenden Aluminium-Zirkonium-Verbindung mit einem mehrwertigen Alkohol bestehen, der 20 - 50 Gew.-%, besonders bevorzugt 20 - 42 Gew.-%, schweißhemmende Aluminium-Zirkonium-Verbindung und 2 - 16 Gew.-% molekular gebundenes Wasser enthält, wobei der Rest zu 100 Gew.-% mindestens eine Verbindung, ausgewählt aus wasserlöslichen mehrwertigen C₂ - C₉-Alkanolen mit 2 - 6 Hydroxyl-gruppen und wasserlöslichen Polyethylenglycolen mit 3 - 20 Ethylenoxid-Einheiten, ist.

Erfindungsgemäß besonders bevorzugte Antitranspirant-Wirkstoffe sind ausgewählt aus so genannten "aktivierten" Aluminium-Zirkonium-Verbindungen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium-Zirkonium-Verbindungen werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium-Zirkonium-Verbindungen haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70 Gew.-% des Aluminiums diesen Peaks zuzuordnen sind.

Sowohl aktivierte als auch nicht-aktivierte Aluminium-Zirkonium-Verbindungen können als getrocknetes, z. B. sprühgetrocknetes, Pulver eingesetzt werden. In einer besonders bevorzugten Ausführungsform wird dieses Pulver als Solubilisat in mindestens einem Polyol, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, eingesetzt. Bevorzugt sind diese wasserlöslichen Polyolkomponenten ausgewählt aus 1,2-Propylenglycol, 1,3-Propylenglycol, 2-Methyl-1,3-propandiol, Glycerin, Butylenglycolen wie bevorzugt 1,2-Butylenglycol, besonders bevorzugt 1,3-Butylenglycol, und 1,4-Butylenglycol, Pentaerythrit, Pentylenglycolen wie 1,2-Pentandiol und 1,5-Pentandiol, Hexandiolen, wie besonders bevorzugt 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 besonders bevorzugt sind. Bevorzugte Solubilisierungsmittel sind 1,2-Propylenglycol, 1,3-Butylenglycol, Dipropylenglycol und 1,2-Hexandiol sowie Mischungen hiervon. Bevorzugte solubilisierende Polyolmischungen enthalten 1,2-Propylenglycol und Dipropylenglycol. Besonders bevorzugte Polyolmischungen enthalten 1,2-Propylenglycol und Dipropylenglycol im Gewichtsverhältnis von 4:1 bis 2:1.

Erfindungsgemäß ebenfalls bevorzugte schweißhemmende Wirkstoffe sind nicht-wässrige Lösungen oder Solubilisate einer aktivierten schweißhemmenden Aluminium-Zirkonium-Verbindung, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen aufweist, bevorzugt Propylenglycol, Sorbit und Pentaerythrit, gegen den Verlust der Aktivierung gegen den raschen Abbau des HPLC-Peak 4 : Peak 3-Flächenverhältnisses der Verbindung stabilisiert sind. Beispielsweise bevorzugt sind Antitranspirant-Wirkstoff-Lösungen oder -Solubilisate (gemeint sind nicht die erfindungsgemäßen Emulsionen), die in Gewichtsprozent (USP) enthalten: 18 - 45 Gew.- % eines aktivierten Aluminium-Zirkoniumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium-Zirconiumsalze mit einem mehrwertigen Alkohol, die 20 - 50 Gew.-%, besonders bevorzugt 20 - 42 Gew.-%, aktiviertes schweißhemmendes Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatomen und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 bis 1:28 und bevorzugt 1:2 bis 1:25 bereitzustellen.

Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.- % molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der I-Form und der dI-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium-Zirconiumsalze, wie sie beispielsweise in US 6902723 offenbart sind, enthaltend 5 - 78 Gew.-% (USP) eines aktivierten schweißhemmenden Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(Al+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein molares Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein molares Al:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1,5 - 1,87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Bevorzugte Aluminiumzirconiumsalze weisen ein molares Metall-zu-Chlorid-Verhältnis von 0,9 - 2,0, bevorzugt 1,0 - 1,51, besonders bevorzugt 1,1 - 1,5, außerordentlich bevorzugt 1,3 - 1,4, auf. Bevorzugte Aluminiumzirconiumchlorohydrate haben die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9).

Weitere bevorzugte Aluminiumzirconiumtrichlorohydrate haben die empirische Formel Al₄(OH)₁₀Cl₂ · Zr(OH)Cl. Weitere bevorzugte Aluminiumzirconiumtetrachlorohydrate haben die empirische Formel Al₄(OH)₁₀Cl₂ · ZrCl₂. Weitere bevorzugte Aluminiumzirconiumpentachlorohydrate haben die empirische Formel Al₈(OH)₂₀Cl₆ · Zr(OH)Cl. Weitere bevorzugte Aluminiumzirconiumoctachlorohydrate haben die empirische Formel Al₈(OH)₁₈Cl₆ · Zr(OH)Cl.

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Bevorzugte schweißhemmende Salze sind Aluminium-Zirconiumtetrachlorohydrate (molares Verhältnis AI:Zr = 2-6; M:CI = 0.9-1.3), insbesondere Salze mit einem molaren Metall-zu-Chlorid-Verhältnis von 0,9 - 1,1, bevorzugt 0,9 - 1,0.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Die vorstehend genannten Aluminium-Zirconiumtrichlorhydrate, Aluminium-Zirconiumtetrachlorhydrate, Aluminium-Zirconiumpentachlorhydrate und Aluminium-Zirconiumoctachlorhydrate liegen - sowohl aktiviert als auch nicht-aktiviert - bevorzugt als Komplex mit Glycin vor.

Besonders bevorzugte schweißhemmende Wirkstoffe sind ausgewählt aus aktiviertem Aluminium-Zirconiumtrichlorohydrex Glycine, insbesondere aktiviertem Aluminium-Zirconiumtrichlorohydrex Glycine mit einer kristallwasserfreien und Glycin-freien Aktivsubstanz (USP) von 69,5 - 88 Gew.- %, bevorzugt 72 - 85 Gew.-%, besonders bevorzugt 77 - 80 Gew.-%, jeweils bezogen auf den Rohstoff tel quel, einem molaren Metall:Cl-Verhältnis von 0,9 bis 1,5 und einem molaren AI:Zr-Verhältnis von 3,4 - 3,8.

Weitere besonders bevorzugte schweißhemmende Wirkstoffe sind ausgewählt aus nicht-aktiviertem Aluminium-Zirconiumtrichlorohydrex Glycine, insbesondere nicht-aktiviertem Aluminium-Zirconiumtrichlorohydrex Glycine mit einer kristallwasserfreien und Glycin-freien Aktivsubstanz (USP) von 69,5 - 88 Gew.-%, bevorzugt 72 - 85 Gew.-%, besonders bevorzugt 77 - 80 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus aktiviertem Aluminium-Zirconiumtetrachlorohydrex Glycine, insbesondere aktiviertem Aluminium-Zirconiumtetrachlorohydrex Glycine mit einer kristallwasserfreien und Glycin-freien Aktivsubstanz (USP) von 72 - 88 Gew.-%, bevorzugt 77 - 85 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8.

Weitere besonders bevorzugte schweißhemmende Wirkstoffe sind ausgewählt aus nicht-aktiviertem Aluminium-Zirconiumtetrachlorohydrex Glycine, insbesondere nicht-aktiviertem Aluminium-Zirconiumtetrachlorohydrex Glycine mit einer kristallwasserfreien und Glycin-freien Aktivsubstanz (USP) von 72 - 88 Gew.-%, bevorzugt 77 - 85 Gew.-%, jeweils bezogen auf den Rohstoff tel quel, einem molaren Metall:Cl-Verhältnis von 0,9 bis 1,5 und einem molaren Al:Zr-Verhältnis von 3,4 - 3,8. Weitere besonders bevorzugte schweißhemmende Wirkstoffe sind ausgewählt aus aktiviertem Aluminium-Zirconiumpentachlorohydrex Glycine, insbesondere aktiviertem Aluminium-Zirconiumpentachlorohydrex Glycine mit einer kristallwasserfreien und Glycin-freien Aktivsubstanz (USP) von 72 - 88 Gew.-%, bevorzugt 77 - 86 Gew.-%, besonders bevorzugt 78 - 81,5 Gew.-%, jeweils bezogen auf den Rohstoff tel quel, einem molaren Metall:Cl-Verhältnis von 1,51 bis 2,0 und einem molaren Al:Zr-Verhältnis von 9,2 - 9,8. Weitere besonders bevorzugte schweißhemmende Wirkstoffe sind ausgewählt aus nicht-aktiviertem Aluminium-Zirconiumpentachlorohydrex Glycine, insbesondere nicht-aktiviertem Aluminium-Zirconiumpentachlorohydrex Glycine mit einer kristallwasserfreien und Glycin-freien Aktivsubstanz (USP) von 72 - 88 Gew.-%, bevorzugt 77 - 86 Gew.-%, besonders bevorzugt 78 - 81,5 Gew.-%, jeweils bezogen auf den Rohstoff tel quel, einem molaren Metall:Cl-Verhältnis von 1,51 bis 2,0 und einem molaren Al:Zr-Verhältnis von 9,2 - 9,8. Der Kristallwassergehalt beträgt bei den vorgenannten aktivierten wie nicht-aktivierten Aluminium-Zirconiumtrichlorohydrex Glycine, Aluminium-Zirconiumtetrachlorohydrex Glycine, Aluminium-Zirconiumpentachlorohydrex Glycine und Aluminium-Zirconiumoctachlorohydrex Glycine 1,5 - 20 Gew.-%, bevorzugt 7 - 15 Gew.-%, jeweils bezogen auf den Rohstoff tel quel.

Weitere bevorzugte Aluminium-Zirconiumtrichlorohydrex Glycine haben die empirische Formel [Al₄(OH)₁₀Cl₂ · Zr(OH)Cl] · NH₂CH₂COOH. Weitere bevorzugte Aluminium-Zirconiumtetrachlorohydrex Glycine haben die empirische Formel [Al₄(OH)₁₀Cl₂ · ZrOCl₂] · NH₂CH₂COOH. Weitere bevorzugte Aluminium-Zirconiumpentachlorohydrex Glycine haben die empirische Formel [Al₈(OH)₂₀Cl₄ · Zr(OH)Cl] · NH₂CH₂COOH. Weitere bevorzugte Aluminium-Zirconiumoctachlorohydrex Glycine haben die empirische Formel [Al₈(OH)₁₈Cl₆ · Zr(OH)Cl] · NH₂CH₂COOH oder [Al₈(OH)₁₈Cl₆ · ZrOCl₂] · NH₂CH₂COOH.

Weiterhin erfindungsgemäß bevorzugt sind Aluminiumzirconiumchlorohydrat-Glycin-Salze, die mit Betain ((CH₃)₃N⁺-CH₂₋COO⁻) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt. Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

Weitere bevorzugte Antitranspirant-Wirkstoffe sind ausgewählt aus adstringierenden Titansalzen, wie sie beispielsweise in GB 2299506 A offenbart sind.

Die Antitranspirant-Wirkstoffe können als nicht-wässrige Lösungen oder als glycolische Solubilisate eingesetzt werden.

Besonders bevorzugt sind Aluminium-Zirkonium-Tetrachlorohydrex-Glycin-Komplexe, die in mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, solubilisiert sind.

Aluminium-Zirkonium-Tetrachlorohydrex-Glycin-Komplexe sind zum Beispiel von Summit Reheis unter der Bezeichnung Rezal® 36 GP oder, in aktivierter Qualität, als Summit Reach® AZP-908, Summit AZG-369 oder Summit AZG-364 als Pulver im Handel.

Weiterhin besonders bevorzugt sind Aluminium-Zirkonium-Pentachlorohydrex-Glycin-Komplexe, die in mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, solubilisiert sind. Solche Aluminium-Zirkonium-Pentachlorohydrex-Glycin-Komplexe sind zum Beispiel in aktivierter Qualität von Summit unter der Bezeichnung AAZG-3110 als Pulver im Handel.

Die wässrige bzw. hydrophile Phase der erfindungsgemäßen Zusammensetzungen umfasst weiterhin mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten. Der Zusatz dieser Verbindungen ist insbesondere nötig, um die Transparenz der gesamten Emulsion zu erreichen. Der Brechungsindex der wässrigen bzw. hydrophilen Phase ohne diese Polyole wäre zu niedrig, um an den Brechungsindex der Ölphase angeglichen werden zu können. Außerdem tragen diese wasserlöslichen Polyolkomponenten zur Stabilität der Emulsion bei.

Bevorzugt sind diese wasserlöslichen Polyolkomponenten ausgewählt aus 1,2-Propylenglycol, 1,3-Propylenglycol, 2-Methyl-1,3-propandiol, Glycerin, Butylenglycolen wie bevorzugt 1,2-Butylenglycol, besonders bevorzugt 1,3-Butylenglycol, und 1,4-Butylenglycol, Pentylenglycolen wie 1,2-Pentandiol und 1,5-Pentandiol, Hexandiolen, wie besonders bevorzugt 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 besonders bevorzugt sind. Auch Zucker, Zuckeralkohole und bestimmte Zuckerderivate wie Pentaerythritol, Erythrit, Sorbit, Xylitol, Fructose, Glucose, Maltose, Maltitol, Mannit, Inosit, Sucrose, Trehalose und Xylose können erfindungsgemäß eingesetzt werden, können jedoch aufgrund ihrer Klebrigkeit weniger geeignet oder nur in geringen Mengen geeignet sein.

Bevorzugt sind 1,2-Propylenglycol, 1,3-Butylenglycol, Dipropylenglycol und 1,2-Hexandiol sowie Mischungen hiervon. Bevorzugte Polyolmischungen enthalten 1,2-Propylenglycol und Dipropylenglycol. Besonders bevorzugte Polyolmischungen enthalten 1,2-Propylenglycol und Dipropylenglycol im Gewichtsverhältnis von 4:1 bis 2:1. Diese Mischungen zeigen in Bezug auf Transparenz, Wirkstofffreisetzung, Hautgefühl und Hautverträglichkeit besonders ausgewogene Eigenschaften. Bevorzugte erfindungsgemäße Zusammensetzungen sind dadurch gekennzeichnet, dass die mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, in einer Gesamtmenge von 20 - 60 Gew.-%, bevorzugt 25 - 55 Gew.-%, besonders bevorzugt 30 - 50 Gew.-%, außerordentlich bevorzugt 35 - 45 Gew.-%, jeweils bezogen auf das Gesamtgewicht der Emulsion, enthalten ist.

Die erfindungsgemäßen Zusammensetzungen umfassen weiterhin mindestens einen Silicon-basierten Wasser-in-Öl-Emulgator mit mindestens einem Alkylsubstituenten R mit 4 - 20 Kohlenstoffatomen.

Überraschend wurde gefunden, dass Wasser-in-Öl-Emulgatoren, die Silicon-basiert sind, eine bessere Produktleistung im Hinblick auf eine schnellere Freisetzung des schweißhemmenden Wirkstoffs ermöglichen. Im Vergleich dazu waren Wasser-in-Öl-Emulsionen, die alle erfindungsgemäß beanspruchten Merkmale bis auf den Silicon-basierten Wasser-in-Öl-Emulgator aufwiesen, aber statt mit einem Silicon-basierten Wasser-in-Öl-Emulgator mit PEG-30 Dipolyhydroxystearate, einem typischen Silicon-freien Wasser-in-Öl-Emulgator, stabilisiert waren, deutlich schlechter in Bezug auf eine schnelle Freisetzung des schweißhemmenden Wirkstoffs.

In einer bevorzugten Ausführungsform sind die erfindungsgemäßen schweißhemmenden Zusammensetzungen frei von PEG-30 Dipolyhydroxystearate.

Weiterhin wurden die Silicon-freien Wasser-in-Öl-Emulgatoren Isolan GPS (Evonik), eine Mischung aus Polyglyceryl-4 Diisostearate, Polyhydroxystearate und Polyhydroxysebacate, weiterhin Laureth-2, PEG-6 Caprylic/Capric Glycerides, PPG-15 Stearyl Ether und PEG-7 Glyceryl Cocoate getestet: Laureth-2 und PEG-7 Glyceryl Cocoate ergaben keine stabilen Emulsionen, die übrigen Silicon-freien Wasser-in-Öl-Emulgatoren ergaben keine verbesserte Wirkstofffreisetzung.

Eine erfindungsgemäß besonders bevorzugte Gruppe von Wasser-in-Öl-Emulgatoren sind die Poly-(C₂-C₃)alkylenglycol-modifizierten Silicone, die mit mindestens einem Alkylsubstituenten R mit 4 - 20 Kohlenstoffatomen im Molekül hydrophob modifiziert sind. Der hydrophobe Alkylsubstituent R mit 4 - 20 Kohlenstoffatomen ist bevorzugt ausgewählt aus n-Butyl, n-Pentyl, n-Hexyl, n-Heptyl, n-Octyl, 2-Ethylhexyl, n-Decyl, n-Undecyl, n-Dodecyl, n-Tetradecyl, n-Hexadecyl = Cetyl, n-Octadecyl = Stearyl und n-Eicosanyl = Arachyl. Außerordentlich bevorzugt ist der Cetylsubstituent. Bevorzugte (C₂-C₃)-Alkylenglycole, mit denen die Silicone so modifiziert sind, dass sie oberflächenaktive Eigenschaften erhalten, sind ausgewählt aus Ethylenglycol und Propylenglycol. Die gewünschten Emulgator-Eigenschaften lassen sich über das Gewichtsverhältnis von Ethylenglycol- und Propylenglycol-Einheiten im Molekül steuern.

Besonders bevorzugte Silicon-basierte Wasser-in-Öl-Emulgatoren sind ausgewählt aus Cetyl PEG/PPG-10/1 Dimethicone (früher: Cetyl Dimethicone Copolyol, erhältlich als Abil EM 90), weiterhin bevorzugt Cetyl PEG/PPG-7/3 Dimethicone (z. B. Hansa SW 3010 (CHT R. Beitlich GmbH), PEG/PPG-10/3 Oleyl Ether Dimethicone, Lauryl Dimethicone PEG-15 Crosspolymer, Cetyl PEG/PPG-15/15 Butyl Ether Dimethicone (z. B. Wacker-Belsil DMC 3071 VP) und Lauryl PEG/PPG-18/18 Methicone (z. B. Dow Corning 5200 Formulation Aid von Dow Corning). Außerordentlich bevorzugt ist Cetyl PEG/PPG-10/1 Dimethicone.

Bevorzugte erfindungsgemäße Zusammensetzungen sind dadurch gekennzeichnet, dass der mindestens eine Silicon-basierte Wasser-in-Öl-Emulgator mit mindestens einem Alkylsubstituenten R mit 4 - 20 Kohlenstoffatomen in einer Gesamtmenge von 1 - 4 Gew.-%, bevorzugt 1,5 - 3,5 Gew.- %, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist.

Weitere erfindungsgemäß bevorzugte schweißhemmende Zusammensetzungen sind dadurch gekennzeichnet, dass Cetyl PEG/PPG-10/1 Dimethicone in einer Gesamtmenge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist. Weitere erfindungsgemäß bevorzugte schweißhemmende Zusammensetzungen sind dadurch gekennzeichnet, dass Lauryl PEG/PPG-18/18 Methicone in einer Gesamtmenge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist. Weitere erfindungsgemäß bevorzugte schweißhemmende Zusammensetzungen sind dadurch gekennzeichnet, dass Cetyl PEG/PPG-15/15 Butyl Ether Dimethicone in einer Gesamtmenge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist. Weitere erfindungsgemäß bevorzugte schweißhemmende Zusammensetzungen sind dadurch gekennzeichnet, dass Cetyl PEG/PPG-7/3 Butyl Ether Dimethicone in einer Gesamtmenge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist.

Weitere erfindungsgemäß bevorzugte schweißhemmende Zusammensetzungen sind dadurch gekennzeichnet, dass Cetyl PEG/PPG-10/1 Dimethicone und PPG-3-Myristylether enthalten sind. Weitere erfindungsgemäß bevorzugte schweißhemmende Zusammensetzungen sind dadurch gekennzeichnet, dass Cetyl PEG/PPG-10/1 Dimethicone in einer Menge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, und PPG-3-Myristylether in einer Menge von 0,8 bis 4 Gew.-%, bevorzugt 1 - 3 Gew.- %, besonders bevorzugt 2 - 2,5 Gew.-%, jeweils bezogen auf die gesamte Zusammensetzung, enthalten sind.

Weitere erfindungsgemäß bevorzugte schweißhemmende Zusammensetzungen sind dadurch gekennzeichnet, dass Lauryl PEG/PPG-18/18 Methicone und PPG-3-Myristylether enthalten sind. Weitere erfindungsgemäß bevorzugte schweißhemmende Zusammensetzungen sind dadurch gekennzeichnet, dass Lauryl PEG/PPG-18/18 Methicone in einer Menge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, und PPG-3-Myristylether in einer Menge von 0,8 bis 4 Gew.-%, bevorzugt 1 - 3 Gew.- %, besonders bevorzugt 2 - 2,5 Gew.-%, jeweils bezogen auf die gesamte Zusammensetzung, enthalten sind. Weitere erfindungsgemäß bevorzugte schweißhemmende Zusammensetzungen sind dadurch gekennzeichnet, dass Cetyl PEG/PPG-15/15 Butyl Ether Dimethicone und PPG-3-Myristylether enthalten sind. Weitere erfindungsgemäß bevorzugte schweißhemmende Zusammensetzungen sind dadurch gekennzeichnet, dass Cetyl PEG/PPG-15/15 Butyl Ether Dimethicone in einer Menge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, und PPG-3-Myristylether in einer Menge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten sind.

Weitere erfindungsgemäß bevorzugte schweißhemmende Zusammensetzungen sind dadurch gekennzeichnet, dass Cetyl PEG/PPG-7/3 Butyl Ether Dimethicone und PPG-3-Myristylether enthalten sind.

Weitere erfindungsgemäß bevorzugte schweißhemmende Zusammensetzungen sind dadurch gekennzeichnet, dass Cetyl PEG/PPG-7/3 Butyl Ether Dimethicone in einer Menge von 1 - 4 Gew.- %, bevorzugt 1,5 - 3,5 Gew.-%, besonders bevorzugt 2 - 3 Gew.-%, außerordentlich bevorzugt 2,2 - 2,5 Gew.-%, und PPG-3-Myristylether in einer Menge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten sind.

Das Gewichtsverhältnis von Silicon-basierten Wasser-in-Öl-Emulgatoren zu Anlagerungsprodukten von 1 bis 14, bevorzugt 1 bis 8, Propylenoxid-Einheiten an ein einwertiges C₃₋₁₆-Alkanol beträgt in einer weiteren bevorzugten Ausführungsform der Erfindung 0,5 - 2, bevorzugt 0,75 - 1,67, besonders bevorzugt 0,9 - 1,33, außerordentlich bevorzugt 1.

Weitere erfindungsgemäß bevorzugte schweißhemmende Zusammensetzungen sind dadurch gekennzeichnet, dass Cetyl PEG/PPG-10/1 Dimethicone und PPG-3-Myristylether in einem Gewichtsverhältnis von 0,5 - 2, bevorzugt 0,75 - 1,67, besonders bevorzugt 0,9 - 1,33, außerordentlich bevorzugt 1, enthalten sind. Weitere erfindungsgemäß bevorzugte schweißhemmende Zusammensetzungen sind dadurch gekennzeichnet, dass Lauryl PEG/PPG-18/18 Methicone und PPG-3-Myristylether in einem Gewichtsverhältnis von 0,5 - 2, bevorzugt von 0,75 - 1,67, besonders bevorzugt von 0,9 - 1,33, außerordentlich bevorzugt von 1, enthalten sind.

Weitere erfindungsgemäß bevorzugte schweißhemmende Zusammensetzungen sind dadurch gekennzeichnet, dass Cetyl PEG/PPG-15/15 Butyl Ether Dimethicone und PPG-3-Myristylether in einem Gewichtsverhältnis von 0,5 - 2, bevorzugt von 0,75 - 1,67, besonders bevorzugt von 0,9 - 1,33, außerordentlich bevorzugt von 1, enthalten sind.

Weitere erfindungsgemäß bevorzugte schweißhemmende Zusammensetzungen sind dadurch gekennzeichnet, dass Cetyl PEG/PPG-7/3 Butyl Ether Dimethicone und PPG-3-Myristylether in einem Gewichtsverhältnis von 0,5 - 2, bevorzugt von 0,75 - 1,67, besonders bevorzugt von 0,9 - 1,33, außerordentlich bevorzugt von 1, enthalten sind.

Zusätzlich zu den oben aufgeführten hydrophob Alkyl-modifizierten Silicon-basierten Wasser-in-ÖI-Emulgatoren können in einer bevorzugten Ausführungsform der Erfindung solche Silicon-basierten Wasser-in-Öl-Emulgatoren enthalten sein, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, weiterhin PEG/PPG-25/25 Dimethicone, erhältlich unter der Bezeichnung Wacker-Belsil DMC 6031 von der Firma Wacker, PEG/PPG-4/12 Dimethicone, das unter der Bezeichnung Abil B 8852 erhältlich ist, Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).

Die erfindungsgemäßen Zusammensetzungen sind auch ohne Silicon-freie Wasser-in-Öl-Emulgatoren stabil und daher frei von ihnen. Es wurde sogar festgestellt, dass einige Silicon-freie Wasser-in-Öl-Emulgatoren die Wirkstofffreisetzung der erfindungsgemäßen Zusammensetzungen negativ beeinflussen können. Beispiele für solche erfindungsgemäß ausgeschlossenen Silicon-freien W/O-Emulgatoren sind ausgewählt aus Substanzen der allgemeinen Formel A-O-(CHR¹-X-CHR²-O-)ₐ-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe >CHOR³ darstellt, R¹ und R² ein Wasserstoffatom oder eine Methylgruppe darstellen und so gewählt werden, dass nicht beide Reste gleichzeitig Methyl darstellen, und R³ ein Wasserstoffatom oder eine verzweigte oder unverzweigte, gesättigte oder ungesättigte Alkyl- oder Acylgruppe mit 1 bis 20 Kohlenstoffatomen darstellt. Weitere ausgeschlossene siliconfreie W/O-Emulgatoren sind so gewählt, dass die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H)_{b}-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.

Weitere ausgeschlossene siliconfreie W/O-Emulgatoren sind ausgewählt aus
(1) gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden;
(2) ethoxylierten Alkoholen und Carbonsäuren mit 8 - 24 C-Atomen, insbesondere mit 16 - 22 C-Atomen, die einen HLB-Wert von 1 - 8 aufweisen, z. B. Laureth-1 oder Laureth-4;
(3) Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind ausgeschlossen.
(4) Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Veresterungsgrad von 1 - 10, vorzugsweise 1 - 5;
(5) Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1 - 10, vorzugsweise 1 - 5;
(6) Propylenglycolestern gesättigter und/oder ungesättigter, verzweigter und/oder unverzweigter Alkancarbonsäuren einer Kettenlänge von 8 - 24, insbesondere 12 - 18 C-Atomen;
(7) Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(8) Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen.

Weitere Beispiele für erfindungsgemäß ausgeschlossene Silicon-freie W/O-Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearyl-ether (Steareth-2), Glycerylsorbitanstearat, Polyglyceryl-4-lsostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyceryl-3-diisostearat, PEG-8 Distearat, Diglycerin Dipolyhydroxystearat, Glycerinisostearat, Sorbitanisostearat, Polyglyceryl-3-methylglucosedistearat, PEG-2 Stearat, PEG-45/Dodecylglycolcopolymer, PEG-22/Dodecylglycolcopolymer und Methoxy PEG- 22/Dodecyl Glycol Copolymer.

Erfindungsgemäße Zusammensetzungen sind dadurch gekennzeichnet, dass mindestens ein Polyethylenglycolether eines linearen oder verzweigten C₁₂ - C₂₂-Alkanols mit 20 - 150 Ethylenoxideinheiten enthalten ist. Diese Verbindungen sind als Öl-in-Wasser-Emulgatoren bekannt. Überraschend wurde festgestellt, dass der Zusatz derartiger Polyethylenglycolether eines linearen oder verzweigten C₁₂ - C₂₂-Alkanols mit 20 - 150 Ethylenoxideinheiten in Kombination mit den übrigen obligatorischen Bestandteilen der erfindungsgemäßen Zusammensetzungen zu einer stark verbesserten, schnelleren Wirkstofffreisetzung des schweißhemmenden Wirkstoffs führt. Besonders überraschend war, dass längst nicht alle Öl-in-Wasser-Emulgatoren diesen begünstigenden Effekt auf die erfindungsgemäßen Zusammensetzungen ausüben, auch nicht solche, die im selben HLB-Wertbereich liegen wie die erfindungsgemäß bevorzugten Polyethylenglycolether eines linearen oder verzweigten C₁₂ - C₂₂-Alkanols mit 20 - 150 Ethylenoxideinheiten. Ohne an diese Theorie gebunden sein zu wollen, wird vermutet, dass bestimmte Öl-in-Wasser-Emulgatoren die Wasser-in-Öl-Struktur der Emulsion störend beeinflussen. Derartige störende Emulgatoren sind erfindungsgemäß weniger oder gar nicht geeignet.

Besonders bevorzugte erfindungsgemäße Zusammensetzungen sind dadurch gekennzeichnet, dass der mindestens eine Polyethylenglycolether eines linearen oder verzweigten C₁₂ - C₂₂-Alkanols mit 20 - 150 Ethylenoxideinheiten ausgewählt ist aus Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, lsoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, Isoceteth-85, Isoceteth-90, Isoceteth-95, Isoceteth-100, lsoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-100, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, sowie Mischungen hiervon. Außerordentlich bevorzugt ist Steareth-100.

Weitere besonders bevorzugte erfindungsgemäße Zusammensetzungen sind dadurch gekennzeichnet, dass der mindestens eine Polyethylenglycolether eines linearen oder verzweigten C₁₂ - C₂₂-Alkanols mit 20 - 150 Ethylenoxideinheiten in einer Gesamtmenge von 1 - 3,5 Gew.-%, bevorzugt 1,5 - 3,0, besonders bevorzugt 1,8 - 2,5 Gew.-%, jeweils bezogen auf die gesamte Zusammensetzung, enthalten ist, wobei auch ein Gehalt an 1,9, 2,0, 2,1, 2,2, 2,3 und 2,4 Gew.-% bevorzugt sein kann.

Weitere erfindungsgemäß bevorzugte Zusammensetzungen sind gekennzeichnet durch eine Viskosität bei 21 °C von 30.000 - 150.000 mPas, bevorzugt 40.000 - 120.000 mPas, besonders bevorzugt 50.000 - 100.000 mPas und außerordentlich bevorzugt 50.000 - 100.000 mPas.

Die Viskositätsangaben beziehen sich auf Messungen mit einem Rotationsviskosimeter der Firma Brookfield unter Auswahl der Spindel und der Umdrehungszahl, wie sie in dem Handbuch "More Solutions to Sticky Problems" der Firma Brookfield empfohlen ist:

Bei Verwendung von T-Spindeln und Helipath:

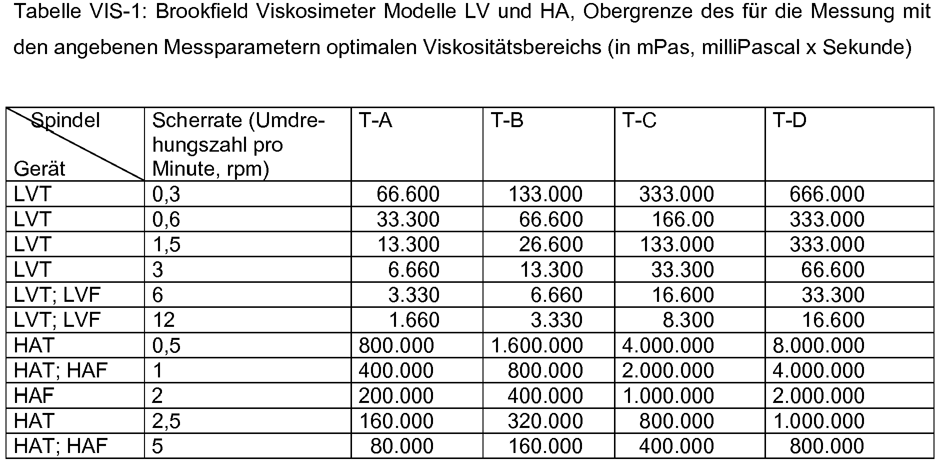

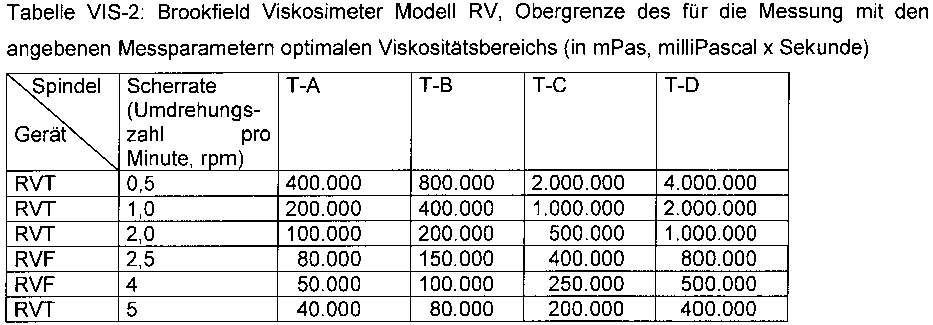

Die angegebene Viskosität stellt den oberen Grenzwert für den optimalen Messbereich für die jeweilige Spindel-Umdrehungszahl-Kombination dar. Falls für einen Viskositätsbereich zwei verschiedene Messparameter-Kombinationen möglich sind, wird die Spindel-Umdrehungszahl-Kombination gewählt, die den höheren Skalenteil-Wert liefert. Weiterhin beziehen sich die Viskositätsangaben auf die Zusammensetzung 24 Stunden nach Herstellung und bei einer Temperatur von 21 °C, gemessen mit einem Helipath.

Erfindungsgemäß bevorzugte Zusammensetzungen mit einer bevorzugten Viskosität sind besonders gut zur Applikation mit einem Kugelapplikator oder aus einem Gelspender geeignet. Um die Transparenz der Zusammensetzung für den Verbraucher deutlich zu machen, ist eine transparente Verpackung bevorzugt.

Weitere erfindungsgemäß bevorzugte Zusammensetzungen können Zusatzstoffe enthalten, wie Deodorantwirkstoffe, UV-Filter, Antioxidantien, desensibilisierende Wirkstoffe, wie Aminosäuren, Proteine und Proteinhydrolysate, Vitamine und Vitaminvorstufen, insbesondere Panthenol, Konservierungsmittel, antibakterielle Wirkstoffe sowie Parfums und Duftstoffe.

Ein weiterer Gegenstand der vorliegenden Anmeldung ist die nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs.

Bezüglich weiterer bevorzugter Ausführungsformen der erfindungsgemäßen Verwendung gilt mutatis mutandis das zu den erfindungsgemäßen Zusammensetzungen Gesagte.

Ein weiterer Gegenstand der vorliegenden Anmeldung ist ein Verfahren zur Herstellung einer schweißhemmenden Zusammensetzung gemäß Anspruch 1, 2, 3, 4, 5, 6, 7, 8, 9, 10, 11 oder 12, wonach die Bestandteile der hydrophoben Ölphase einschließlich des/der Wasser-in-öl-Emulgator/en und des Parfüms miteinander vermischt werden, getrennt hiervon die Bestandteile der hydrophilen Phase, gegebenenfalls inclusive des/der Öl-in-Wasser-Emulgators/en, miteinander vermischt werden, die Brechungsindices n_{D} der beiden Phasen auf eine Differenz von ± 0,0005 bei 25 °C einander angeglichen werden, die Wasserphase unter mäßigem Rühren langsam in die Ölphase eingetropft und die Mischung homogenisiert wird.

Bezüglich weiterer bevorzugter Ausführungsformen der erfindungsgemäßen Verfahren gilt mutatis mutandis das zu den erfindungsgemäßen Zusammensetzungen Gesagte.

Die nachstehenden Beispiele sollen die Erfindung verdeutlichen, ohne sie hierauf zu beschränken.

Transparente schweißhemmende Wasser-in-Öl-Emulsionsgele (alle Mengenangaben in Gew.-%)

| | Nr. 1 | Nr. 2 | Nr 2a | Nr. 3 | Nr. 4 | Nr. 5 | Nr. 6 | Nr. 7 | Nr. 8 | Nr. 9 |
|---|---|---|---|---|---|---|---|---|---|---|
| Diethylhexylcarbonat | 12 | - | 13 | 14 | 14 | 5 | 13 | 12 | 14 | 14 |
| Di-(n-octyl)carbonat | - | 12 | - | - | - | - | - | - | - | - |
| C₁₀-C₁₃-Isoalkan | - | - | - | - | - | 9 | - | - | - | - |
| Cetyl PEG/PPG-10/1 Dimethicone | 2 | 2 | 2,5 | 2,5 | 2,5 | 2,5 | 2,5 | 2,5 | 2,5 | 2,5 |
| PPG-3 Myristylether | 2 | 2 | 1 | - | - | - | 1 | - | - | - |
| Isolan GPS* | - | - | - | - | 0,25 | - | - | - | - | - |
| Softigen 767*** | - | - | - | - | - | - | - | 2 | - | - |
| Ethanol (96 Vol.-%) | 5 | 5 | 5 | 5 | 5 | 5 | 5 | 5 | 5 | 5 |
| Parfümöl | 0,6 | 0,6 | 0,6 | 0,6 | 0,6 | 0,6 | 0,6 | 0,6 | 0,6 | 0,6 |
| Aluminium Zirconium Tetrachlorohydrex Gly (nicht aktiviert), als 46 Gew.-%ige wässrige Lösung vorliegend (Mengenangabe nach USP) | 8,3 | 8,3 | 8,3 | 9,7 | 8,3 | 8,3 | 8,3 | 8,3 | 8,3 | 8,3 |
| Aluminium Zirconium Tetrachlorohydrex Gly (aktiviert), als Komplex mit 1,2-Propylenglycol in 1,2-Propylenglycol solubilisiert vorliegend (Mengenangabe nach USP) | 8,1 | 8,1 | 8,1 | 7,3 | 8,1 | 8,1 | 8,1 | 8,1 | 8,1 | 8,1 |
| | Nr. 1 | Nr. 2 | Nr.2A | Nr. 3 | Nr. 4 | Nr. 5 | Nr. 6 | Nr. 7 | Nr. 8 | Nr. 9 |
| Glycin + Natriumglycinat (aus den beiden schweißhemmenden Wirkstoffen) | 4,2 | 4,2 | 4,2 | 4,3 | 4,2 | 4,2 | 4,2 | 4,2 | 4,2 | 4,2 |
| 1,2-Propylenglycol | 24,4 | 24,4 | 24,4 | 21,8 | 24,4 | 24,4 | 24,4 | 24,4 | 24,4 | 24,4 |
| Dipropylenglycol | 13,4 | 13,4 | 11 | 17,9 | 16,8 | 13,4 | 16,9 | 15,0 | 10,9 | 11,4 |
| Steareth-100 | 2 | 2 | 2 | - | - | - | - | - | - | - |
| Antil 200** | - | - | - | - | - | 2 | - | - | - | - |
| PEG-100 Stearate | - | - | - | - | - | - | - | - | 2 | - |
| PEG-150 Stearate | - | - | - | - | - | - | - | - | - | 2 |
| freies Wasser und molekular gebundenes Wasser | ad 100 | ad 100 | ad 100 | ad 100 | ad 100 | ad 100 | ad 100 | ad 100 | ad 100 | ad 100 |
| Freisetzung des schweißhemmenden Wirkstoffs (Steigung der Leitfähigkeits-Zeit-Kurve) Skala 1 - 5 (gutschlecht) | 2 | 2 | 1 | 5 | 3 | 3 | 3 | 4 | 4 | 4 |

| | | | | | | | | | | |
|---|---|---|---|---|---|---|---|---|---|---|
| * Isolan GPS (Evonik): INCI: Polyglyceryl-4 Diisostearate/Polyhydroxystearate/Sebacate ** Antil 200 (Evonik): INCI: PEG-200 Hydrogenated Glyceryl Palmate, PEG-7 Glyceryl Cocoate *** Softigen 767 (Sasol): INCI: PEG-6 Caprylic/Capric Glycerides | | | | | | | | | | |

Die Beispiele Nr. 1, Nr. 2 und Nr. 2a sind erfindungsgemäß, bei den Beispielen Nr. 3 - 9 handelt es sich um nicht erfindungsgemäße Vergleichsbeispiele.

Die schweißhemmenden Zusammensetzungen Nr. 1 bis Nr. 9 wurden hergestellt, indem die Bestandteile der hydrophoben Ölphase einschließlich dem/den Wasser-in-ÖI-Emulgator/en und dem Parfüm miteinander vermischt wurden, getrennt hiervon die Bestandteile der hydrophilen Phase, gegebenenfalls inclusive des/der Öl-in-Wasser-Emulgators/en, miteinander vermischt wurden, die Brechungsindices n_{D} der beiden Phasen auf eine Differenz von ± 0,0005 bei 25 °C einander angeglichen wurden, die Wasserphase unter mäßigem Rühren langsam in die Ölphase eingetropft und die Mischung homogenisiert wurde. Alle Beispielzusammensetzungen waren hoch transparent und zeigten eine gute Lagerstabilität (12 Wochen bei 40 °C).

Mit einem transparenten Rollon oder Gelspender wurden diese transparenten schweißhemmenden Wasser-in-ÖI-Emulsionsgele auf die Haut im Achselbereich aufgetragen.

## Patentansprüche

1. Transparente schweißhemmende Zusammensetzung in Form einer Wasser-in-Öl-Emulsion, enthaltend
a) 10 - 20 Gew.-% äußere Ölphase, enthaltend
a.i) mindestens einen symmetrischen, unsymmetrischen oder cyclischen Ester der Kohlensäure mit linearen oder verzweigten C₆ - C₂₂-Alkanolen,
a.ii) mindestens ein Anlagerungsprodukt von 1 - 14 Propylenoxid-Einheiten an ein- oder mehrwertige C₃₋₁₆-Alkanole sowie
a.iii) Cyclomethicone in einer Gesamtmenge von 0 bis maximal 3 Gew.-%,
b) 75 - 90 Gew.-% dispergierte wässrige Phase, darin
b.i) mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, in einer Gesamtmenge von 20 - 60 Gew.-%,
b.ii) 5 - 40 Gew.-% (USP) mindestens einer schweißhemmenden Aluminium-Zirkonium-Verbindung, und
b.iii) freies (= nicht molekular gebundenes) und molekular gebundenes Wasser in einer Gesamtmenge von 5 - 40 Gew.-%, bevorzugt 6 - 30 Gew.-%, besonders bevorzugt 7 - 22 Gew.-%,
c) mindestens einen Silicon-basierten Wasser-in-ÖI-Emulgator mit mindestens einem Alkylsubstituenten R mit 4 - 20 Kohlenstoffatomen,
d) keinen Silicon-freien Wasser-in-Öl-Emulgator, wobei der Begriff Silicon-freier Wasser-in-Öl-Emulgator Laureth-2, PEG-6 Caprylic/Capric Glycerides, PPG-15 Stearyl Ether, PEG-7 Glyceryl Cocoate sowie einer Mischung aus Polyglyceryl-4 Diisostearate, Polyhydroxystearate und Polyhydroxysebacate einschließt,
e) mindestens einen Polyethylenglycolether eines linearen oder verzweigten C₁₂ - C₂₂-Alkanols mit 20 - 150 Ethylenoxideinheiten,
wobei alle Gew.-%-Angaben auf das Gesamtgewicht der Emulsion bezogen sind, **dadurch gekennzeichnet, dass** mindestens 20 Gew.-% der mindestens einen schweißhemmenden Aluminium-Zirkonium-Verbindung, bezogen auf die Gesamtmenge an schweißhemmender Aluminium-Zirkonium-Verbindung, solubilisiert sind in mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten.

2. Schweißhemmende Zusammensetzung gemäß Anspruch 1, **dadurch gekennzeichnet, dass** 20 - 100 Gew.-% der mindestens einen schweißhemmenden Aluminium-Zirkonium-Verbindung aus mindestens einem Komplex einer schweißhemmenden Aluminium-Zirkonium-Verbindung mit einem mehrwertigen Alkohol bestehen, der 20 - 50 Gew.-%, besonders bevorzugt 20 - 42 Gew.-%, schweißhemmende Aluminium-Zirkonium-Verbindung und 2 - 16 Gew.-% molekular gebundenes Wasser enthält, wobei der Rest zu 100 Gew.-% mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, ist.

3. Schweißhemmende Zusammensetzung gemäß Anspruch 1 oder 2, **dadurch gekennzeichnet, dass** die mindestens eine schweißhemmende Aluminium-Zirkonium-Verbindung aktiviert ist.

4. Zusammensetzung gemäß Anspruch 1, 2 oder 3, **dadurch gekennzeichnet, dass** die mindestens eine schweißhemmende Aluminium-Zirkonium-Verbindung in einer Gesamtmenge von 8 - 25 Gew.-%, bevorzugt 10 - 22 und besonders bevorzugt 13 - 20 Gew.-%, bezogen auf den Aktivsubstanzgehalt nach US Pharmacopeia (USP) in der gesamten Zusammensetzung, enthalten ist.

5. Schweißhemmende Zusammensetzung gemäß Anspruch 1, 2, 3 oder 4, **dadurch gekennzeichnet, dass** der Silicon-basierte Wasser-in-Öl-Emulgator ausgewählt ist aus Poly-(C₂-C₃)-alkylen-glycol-modifizierten Siliconen, die mit mindestens einem Alkylsubstituenten R mit 4 - 20 Kohlenstoffatomen im Molekül hydrophob modifiziert sind.

6. Schweißhemmende Zusammensetzung gemäß Anspruch 1, 2, 3, 4 oder 5, **dadurch gekennzeichnet, dass** der Silicon-basierte Wasser-in-Öl-Emulgator ausgewählt ist aus Cetyl PEG/PPG-10/1 Dimethicone, Cetyl PEG/PPG-7/3 Dimethicone, PEG/PPG-10/3 Oleyl Ether Dimethicone, Lauryl Dimethicone PEG-15 Crosspolymer, Cetyl PEG/PPG-15/15 Butyl Ether Dimethicone und Lauryl PEG/PPG-18/18 Methicone.

7. Zusammensetzung gemäß Anspruch 1, 2, 3, 4, 5 oder 6, **dadurch gekennzeichnet, dass** der mindestens eine Silicon-basierte Wasser-in-Öl-Emulgator mit mindestens einem Alkylsubstituenten R mit 4 - 20 Kohlenstoffatomen in einer Gesamtmenge von 1 - 4 Gew.-%, bevorzugt 1,5 - 3,5 Gew.-%, besonders bevorzugt 2 - 3 Gew.-%, außerordentlich bevorzugt 2,2 - 2,5 Gew.-%, jeweils bezogen auf das Gesamtgewicht der Zusammensetzung, enthalten ist.

8. Zusammensetzung gemäß Anspruch 1, 2, 3, 4, 5, 6 oder 7, **dadurch gekennzeichnet, dass** das Anlagerungsprodukt von 1 - 14 Propylenoxid-Einheiten an ein- oder mehrwertige C₃₋₁₆-Alkanole ausgewählt ist aus PPG-2-Myristylether und PPG-3-Myristylether, bevorzugt ausgewählt aus PPG-3-Myristylether.

9. Zusammensetzung gemäß Anspruch 1, 2, 3, 4, 5, 6, 7 oder 8, **dadurch gekennzeichnet, dass** das mindestens eine Anlagerungsprodukt von 1 - 14, bevorzugt 1 - 8, Propylenoxid-Einheiten an ein ein- oder mehrwertiges C₃₋₁₆-Alkanol, bevorzugt an ein einwertiges C₁₀₋₁₄-Alkanol, in einer Gesamtmenge von 0,8 bis 4 Gew.-%, bevorzugt 1 - 3 Gew.-%, besonders bevorzugt 2 - 2,5 Gew.-%, jeweils bezogen auf die gesamte Zusammensetzung, enthalten ist.

10. Zusammensetzung gemäß Anspruch 1, 2, 3, 4, 5, 6, 7, 8 oder 9, **dadurch gekennzeichnet, dass** die hydrophobe Ölphase ohne die Emulgatoren zu mindestens 60 Gew.-%, bevorzugt zu mindestens 70 Gew.-%, besonders zu mindestens 80 Gew.-%, außerordentlich bevorzugt zu mindestens 90 Gew.-%, aus mindestens einem symmetrischen, unsymmetrischen oder cyclischen Ester der Kohlensäure mit linearen oder verzweigten C₆ - C₂₂-Alkanolen besteht.

11. Zusammensetzung gemäß Anspruch 1, 2, 3, 4, 5, 6, 7, 8, 9 oder 10, **dadurch gekennzeichnet, dass** die mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten, in einer Gesamtmenge von 25 - 55 Gew.-%, besonders bevorzugt 30 - 50 Gew.-%, außerordentlich bevorzugt 35 - 45 Gew.-%, jeweils bezogen auf das Gesamtgewicht der Emulsion, enthalten ist.

12. Zusammensetzung gemäß Anspruch 1, **dadurch gekennzeichnet, dass** lineare Polymethylsiloxane oder Polydimethylsiloxane mit einer kinematischen Viskosität bei 25 °C von 20 cSt und darunter in einer Gesamtmenge von 0 bis maximal 3 Gew.-% bevorzugt 0 bis maximal 2,5 Gew.-%, besonders bevorzugt 0 bis maximal 2 Gew.-%, außerordentlich bevorzugt 0 bis maximal 1 Gew.-%, enthalten sind.

13. Nicht-therapeutische, kosmetische Verwendung einer schweißhemmenden Zusammensetzung gemäß Anspruch 1, 2, 3, 4, 5, 6, 7, 8, 9, 10, 11 oder 12 zur Reduzierung und/oder Regulierung der Transpiration und/oder des Körpergeruchs.

14. Verfahren zur Herstellung einer schweißhemmenden Zusammensetzung gemäß Anspruch 1, 2, 3, 4, 5, 6, 7, 8, 9, 10, 11 oder 12, **dadurch gekennzeichnet, dass** die Bestandteile der hydrophoben Ölphase einschließlich des/der Wasser-in-Öl-Emulgator/en und des Parfüms miteinander vermischt werden, getrennt hiervon die Bestandteile der hydrophilen Phase, gegebenenfalls inclusive des/der Öl-in-Wasser-Emulgators/en, miteinander vermischt werden, die Brechungsindices n_{D} der beiden Phasen auf eine Differenz von ± 0,0005 bei 25 °C einander angeglichen werden, die Wasserphase unter mäßigem Rühren langsam in die Ölphase eingetropft und die Mischung homogenisiert wird.

## Claims

1. A transparent antiperspirant composition in the form of a water-in-oil emulsion, containing
a) 10-20 wt.% outer oil phase, containing
a.i) at least one symmetrical, asymmetrical or cyclic ester of carbonic acid with linear or branched C₆-C₂₂ alkanols,
a.ii) at least one addition product of 1-14 propylene oxide units with monovalent or polyvalent C₃₋₁₆ alkanols and
a.iii) cyclomethicone in a total amount of 0 to at most 3 wt.%,
b) 75-90 wt.% dispersed aqueous phase, therein
b.i) at least one compound, selected from water-soluble polyvalent C₂-C₉ alkanols having 2-6 hydroxyl groups and water-soluble polyethylene glycols having 3-20 ethylene oxide units, in a total amount of from 20-60 wt.%,
b.ii) 5-40 wt.% (USP) of at least one antiperspirant aluminum-zirconium compound, and
b.iii) free (= not molecularly bound) and molecularly bound water in a total amount of from 5-40 wt.%, preferably 6-30 wt.%, particularly preferably 7-22 wt.%,
c) at least one silicone-based water-in-oil emulsifier having at least one alkyl substituent R having 4-20 carbon atoms,
d) no silicone-free water-in-oil emulsifier, the term silicone-free water-in-oil emulsifier including laureth-2, PEG-6 caprylic/capric glycerides, PPG-15 stearyl ethers, PEG-7 glyceryl cocoates and a mixture of polyglyceryl-4 diisostearates, polyhydroxy stearates and polyhydroxy sebacates,
e) at least one polyethylene glycol ether of a linear or branched C₁₂-C₂₂ alkanol having 20-150 ethylene oxide units,
all wt.% amounts being based on the total weight of the emulsion, **characterized in that** at least 20 wt.% of the at least one antiperspirant aluminum-zirconium compound, based on the total amount of antiperspirant aluminum-zirconium compound, is solubilized in at least one compound, selected from water-soluble polyvalent C₂-C₉ alkanols having 2-6 hydroxyl groups and water-soluble polyethylene glycols having 3-20 ethylene oxide units.

2. The antiperspirant composition according to claim 1, **characterized in that** 20-100 wt.% of the at least one antiperspirant aluminum-zirconium compound consists of at least one complex of an antiperspirant aluminum-zirconium compound having a polyvalent alcohol, which contains 20-50 wt.%, particularly preferably 20-42 wt.%, antiperspirant aluminum-zirconium compound and 2-16 wt.% molecularly bound water, the remainder to 100 wt.% being at least one compound, selected from water-soluble polyvalent C₂-C₉ alkanols having 2-6 hydroxyl groups and water-soluble polyethylene glycols having 3-20 ethylene oxide units.

3. The antiperspirant composition according to claim 1 or 2, **characterized in that** the at least one antiperspirant aluminum-zirconium compound is activated.

4. The composition according to claim 1, 2 or 3, **characterized in that** the at least one antiperspirant aluminum-zirconium compound is contained in a total amount of from 8-25 wt.%, preferably 10-22 and particularly preferably 13-20 wt.%, based on the active substance content according to US Pharmacopeia (USP) in the total composition.

5. The antiperspirant composition according to claim 1, 2, 3 or 4, **characterized in that** the silicone-based water-in-oil emulsifier is selected from poly(C₂-C₃)alkylene glycol-modified silicones which are hydrophobically modified by at least one alkyl substituent R having 4-20 carbon atoms in the molecule.

6. The antiperspirant composition according to claim 1, 2, 3, 4 or 5, **characterized in that** the silicone-based water-in-oil emulsifier is selected from cetyl PEG/PPG-10/1 dimethicone, cetyl PEG/PPG-7/3 dimethicone, PEG/PPG-10/3 oleyl ether dimethicone, lauryl dimethicone PEG-15 crosspolymer, cetyl PEG/PPG-15/15 butyl ether dimethicone and lauryl PEG/PPG-18/18 methicone.

7. The composition according to claim 1, 2, 3, 4, 5 or 6, **characterized in that** the at least one silicone-based water-in-oil emulsifier having at least one alkyl substituent R having 4-20 carbon atoms is contained in a total amount of from 1-4 wt.%, preferably 1.5-3.5 wt.%, particularly preferably 2-3 wt.%, extremely preferably 2.2-2.5 wt.%, in each case based on the total weight of the composition.

8. The composition according to claim 1, 2, 3, 4, 5, 6 or 7, **characterized in that** the addition product of 1-14 propylene oxide units with monovalent or polyvalent C₃₋₁₆ alkanols is selected from PPG-2-myristyl ethers and PPG-3-myristil ethers, preferably selected from PPG-3-myristyl ethers.

9. The composition according to claim 1, 2, 3, 4, 5, 6, 7 or 8, **characterized in that** the at least one addition product of 1-14, preferably 1-8, propylene oxide units with a monovalent or polyvalent C₃₋₁₆ alkanol, preferably with a monovalent C₁₀₋₁₄ alkanol, is contained in a total amount of from 0.8-4 wt.%, preferably 1-3 wt.%, particularly preferably 2-2.5 wt.%, in each case based on the total composition.

10. The composition according to claim 1, 2, 3, 4, 5, 6, 7, 8 or 9, **characterized in that** the hydrophobic oil phase without the emulsifiers consists of at least 60 wt.%, preferably at least 70wt.%, especially at least 80 wt.%, extremely preferably at least 90 wt.%, at least one symmetrical, asymmetrical or cyclic ester of carbonic acid having linear or branched C₆-C₂₂ alkanols.

11. The composition according to claim 1, 2, 3, 4, 5, 6, 7, 8, 9 or 10, **characterized in that** the at least one compound, selected from water-soluble polyvalent C₂-C₉ alkanols having 2-6 hydroxyl groups and water-soluble polyethylene glycols having 3-20 ethylene oxide units, is contained in a total amount of from 25-55 wt.%, particularly preferably 30-50 wt.%, extremely preferably 35-45 wt.%, in each case based on the total weight of the emulsion.

12. The composition according to claim 1, **characterized in that** linear polymethylsiloxanes or polydimethylsiloxanes having a kinematic viscosity at 25 °C of 20 cSt and below are contained in a total amount of from 0 to at most 3 wt.%, preferably 0 to at most 2.5 wt.%, particularly preferably 0 to at most 2 wt.%, extremely preferably 0 to at most 1 wt.%.

13. The non-therapeutic cosmetic use of an antiperspirant composition according to claim 1, 2, 3, 4, 5, 6, 7, 8, 9, 10, 11 or 12 for reducing and/or regulating sweating and/or body odor.

14. A method for preparing an antiperspirant composition according to claim 1, 2, 3, 4, 5, 6, 7, 8, 9, 10, 11 or 12, **characterized in that** the components of the hydrophobic oil phase including the water-in-oil emulsifier(s) and the perfume are intermixed, the components of the hydrophilic phase, optionally including the oil-in-water emulsifier(s), are intermixed separately therefrom, the refractive indices n_{D} of the two phases are adjusted to one another to a difference of ± 0.0005 at 25 °C, the water phase is slowly added dropwise into the oil phase while gently stirring and the mixture is homogenized.

## Revendications

1. Composition transparente anti-transpirante sous forme d'émulsion eau-dans-huile, contenant
a) 10 à 20 % en poids de phase externe huileuse, contenant
a.i) au moins un ester symétrique, dissymétrique ou cyclique de l'acide carbonique avec des alcanols en C₆-C₂₂ linéaires ou ramifiés,
a.ii) au moins un produit d'addition de 1 à 14 unités d'oxyde de propylène sur des alcanols en C₃-C₁₆ monovalents ou plurivalents ainsi que
a.iii) des cyclométhicones en une quantité totale de 0 à 3 % en poids maximum,
b) 75 à 90 % en poids de phase aqueuse dispersée, et dans cette dernière
b.i) au moins un composé, choisi parmi des alcanols en C₂-C₉ plurivalents solubles dans l'eau comportant 2 à 6 groupes hydroxyle et des polyéthylène glycols solubles dans l'eau comportant 3 à 20 unités d'oxyde d'éthylène, en une quantité totale de 20 à 60 % en poids,
b.ii) 5 à 40 % en poids (USP) d'au moins un composé d'aluminium-zirconium anti-transpirant, et
b.iii) de l'eau libre (= non liée moléculairement) et de l'eau liée moléculairement en une quantité totale de 5 à 40 % en poids, de préférence de 6 à 30 % en poids, de manière particulièrement préférée de 7 à 22 % en poids,
c) au moins un émulsifiant eau-dans-huile à base de silicone comportant au moins un substituant alkyle R doté de 4 à 20 atomes de carbone,
d) aucun émulsifiant eau-dans-huile exempt de silicone, le terme d'émulsifiant eau-dans-huile exempt de silicone incluant le laureth-2, les glycérides capryliques/capriques PEG-6 (PEG-6 Caprylic/Capric Glycerides), l'éther stéarylique PPG-15 (PPG-15 Stearyl Ether), le cocoate de glycéryle PEG-7 (PEG-7 Glyceryl Cocoate) et un mélange de polyglycéryl-4 diisostéarates, de polyhydroxystéarates et de polyhydroxysébacates,
e) au moins un éther de polyéthylène glycol et d'un alcanol en C₁₂-C₂₂ linéaire ou ramifié comportant 20 à 150 unités d'oxyde d'éthylène,
toutes les données exprimées en pourcentage en poids étant basées sur le poids total de l'émulsion, **caractérisée en ce qu'**au moins 20 % en poids de l'au moins un composé d'aluminium-zirconium anti-transpirant, par rapport à la quantité totale de composé d'aluminium-zirconium anti-transpirant, sont solubilisés dans au moins un composé choisi parmi des alcanols en C₂-C₉ plurivalents solubles dans l'eau comportant 2 à 6 groupes hydroxyle et des polyéthylène glycols solubles dans l'eau comportant 3 à 20 unités d'oxyde d'éthylène.

2. Composition anti-transpirante selon la revendication 1, **caractérisée en ce que** 20 à 100 % en poids de l'au moins un composé d'aluminium-zirconium anti-transpirant est constitué d'au moins un complexe d'un composé d'aluminium-zirconium anti-transpirant avec un alcool plurivalent, qui contient 20 à 50 % en poids, de manière particulièrement préférée 20 à 42 % en poids, de composé d'aluminium-zirconium anti-transpirant et 2 à 16 % en poids d'eau liée moléculairement, le complément à 100 % étant au moins un composé, choisi parmi des alcanols en C₂-C₉ plurivalents solubles dans l'eau comportant 2 à 6 groupes hydroxyle, et des glycols de polyéthylène solubles dans l'eau comportant 3 à 20 unités d'oxyde d'éthylène.

3. Composition anti-transpirante selon les revendications 1 ou 2, **caractérisée en ce que** l'au moins un composé d'aluminium-zirconium anti-transpirant est activé.

4. Composition anti-transpirante selon les revendications 1, 2 ou 3, **caractérisée en ce que** l'au moins un composé d'aluminium-zirconium anti-transpirant est présent en une quantité totale de 8 à 25 % en poids, de préférence de 10 à 22 et de manière particulièrement préférée de 13 à 20 % en poids, par rapport à la teneur en substance active selon US Pharmacopeia (USP) dans la composition totale.

5. Composition anti-transpirante selon les revendications 1, 2, 3 ou 4, **caractérisée en ce que** l'émulsifiant eau-dans-huile à base de silicone est choisi parmi des silicones modifiées par polyalkylène-glycol-(en C₂-C₃), qui sont modifiées hydrophobiquement dans la molécule par au moins un substituant alkyle R comportant 4 à 20 atomes de carbone.

6. Composition anti-transpirante selon les revendications 1, 2, 3, 4 ou 5, **caractérisée en ce que** l'émulsifiant eau-dans-huile à base de silicone est choisi parmi Cetyl PEG/PPG- 10/1 Dimethicone, Cetyl PEG/PPG-7/3 Dimethicone, PEG/PPG-10/3 Oleyl Ether Dimethicone, Lauryl Dimethicone PEG-15 Crosspolymer, Cetyl PEG/PPG-15/15 Butyl Ether Dimethicone et Lauryl PEG/PPG-18/18 Methicone.

7. Composition selon les revendications 1, 2, 3, 4, 5 ou 6, **caractérisée en ce que** l'au moins un émulsifiant eau-dans-huile à base de silicone comportant au moins un substituant alkyle R doté de 4 à 20 atomes de carbone est présent en une quantité totale de 1 à 4 % en poids, de préférence de 1,5 à 3,5 % en poids, de manière particulièrement préférée de 2 à 3 % en poids, de manière préférée entre toutes de 2,2 à 2,5 % en poids, dans chaque cas par rapport au poids total de la composition.

8. Composition selon les revendications 1, 2, 3, 4, 5, 6 ou 7, **caractérisée en ce que** le produit d'addition de 1 à 14 unités d'oxyde de propylène sur des alcanols en C₃-C₁₆ monovalents ou plurivalents est choisi parmi l'éther myristylique PPG-2 et l'éther myristylique PPG-3, de préférence choisi à partir de l'éther myristylique PPG-3.

9. Composition selon les revendications 1, 2, 3, 4, 5, 6, 7 ou 8, **caractérisée en ce que** l'au moins un produit d'addition de 1 à 14, de préférence de 1 à 8, unités d'oxyde de propylène sur un alcanol en C₃-C₁₆ monovalent ou plurivalent, de préférence sur un alcanol en C₁₀-C₁₄ monovalent, est présent en une quantité totale allant de 0,8 à 4 % en poids, de préférence de 1 à 3 % en poids, de manière particulièrement préférée de 2 à 2,5 % en poids, dans chaque cas par rapport à la composition totale.

10. Composition selon les revendications 1, 2, 3, 4, 5, 6, 7, 8 ou 9, **caractérisée en ce que** la phase huileuse hydrophobe sans les émulsifiants est constituée à au moins 60 % en poids, de préférence à au moins 70 % en poids, en particulier à au moins 80 % en poids, de manière préférée entre toutes à au moins 90 % en poids d'au moins un ester symétrique, dissymétrique ou cyclique de l'acide carbonique avec des alcanols en C₆-C₂₂ linéaires ou ramifiés.

11. Composition selon les revendications 1, 2, 3, 4, 5, 6, 7, 8, 9 ou 10, **caractérisée en ce que** l'au moins un composé, choisi parmi des alcanols en C₂-C₉ plurivalents solubles dans l'eau comportant 2 à 6 groupes hydroxyle et des polyéthylène glycols solubles dans l'eau comportant 3 à 20 unités d'oxyde d'éthylène, est présent en une quantité totale allant de 25 à 55 % en poids, de manière particulièrement préférée de 30 à 50 % en poids, de manière préférée entre toutes de 35 à 45 % en poids, dans chaque cas par rapport au poids total de l'émulsion.

12. Composition selon la revendication 1, **caractérisée en ce que** des polyméthylsiloxanes ou des polydiméthylsiloxanes linéaires ayant une viscosité cinématique à 25 °C inférieure ou égale à 20 cSt sont présents en une quantité totale de 0 à maximum 3 % en poids, de préférence de 0 à maximum 2,5 % en poids, de manière particulièrement préférée de 0 à maximum 2 % en poids, de manière préférée entre toutes de 0 à maximum 1 % en poids.

13. Utilisation cosmétique, non thérapeutique d'une composition anti-transpirante selon les revendications 1, 2, 3, 4, 5, 6, 7, 8, 9, 10, 11 ou 12, pour la réduction et/ou la régulation de la transpiration et/ou des odeurs corporelles.

14. Procédé de production d'une composition anti-transpirante selon les revendications 1, 2, 3, 4, 5, 6, 7, 8, 9, 10, 11 ou 12, **caractérisé en ce que** les composants de la phase huileuse hydrophobe, y compris du ou des émulsifiants eau-dans-huile et du parfum, sont mélangés les uns aux autres, et que, séparément, les composants de la phase hydrophile, le cas échéant y compris du ou des émulsifiants huile-dans-eau, sont mélangés les uns aux autres, les indices de réfractions n_{D} des deux phases sont ajustés l'un par rapport à l'autre à une différence de ± 0,0005 à 25 °C, la phase aqueuse est progressivement versée goutte-à-goutte dans la phase huileuse sous agitation modérée et le mélange est homogénéisé.
